# EUROPÄISCHE PATENTANMELDUNG

(11) **EP 4 286 530 A1**
(43) Veröffentlichungstag der Anmeldung: **06.12.2023**
(21) Anmeldenummer: 23161603.8
(22) Anmeldetag: 13.03.2023
(51) Int. Cl.: C12Q 1/6806, C12Q 1/689

(54) **VERFAHREN UND KIT ZUM GLEICHZEITIGEN NACHWEIS EINER MEHRZAHL UNTERSCHIEDLICHER PATHOGENE IN EINER PROBE**

(30) Priorität: 30.05.2022 DE 102022113596
(71) Anmelder: Xebios Diagnostics Group GmbH, 40597 Düsseldorf (DE)
(72) Erfinder: Decker, Ludwig, Dr., 9761VB Eelde (NL)
(74) Vertreter: Kluin Patent

(57) **Zusammenfassung**

Es wird ein Verfahren zum Nachweis einer Mehrzahl unterschiedlicher Pathogene in einer Probe vorgeschlagen, wobei in einem Voranreicherungsschritt (1) die Probe zum Voranreichern in ein Voranreicherungsmedium gegeben wird, in einem Lyseschritt (2) Zellwände der Pathogene in der vorangereicherten Probe aufgebrochen werden und somit ein Lysat hergestellt wird, in einem Nachweisschritt (3) mittels einer Multiplex-PCR-Untersuchung die Mehrzahl der Pathogene nachgewiesen wird, wobei der Voranreicherungsschritt (1), der Lyseschritt (2) und der Nachweisschritt (3) in dieser Reihenfolge durchlaufen werden und wobei die Lyse ohne eine Erhöhung der Temperatur vor dem Nachweisschritt (3) abgebrochen wird. Weiterhin wird ein Kit (100) zum Nachweis einer Mehrzahl unterschiedlicher Pathogene vorgeschlagen.

## Beschreibung

Die Erfindung betrifft ein Verfahren zum gleichzeitigen Nachweis einer Mehrzahl unterschiedlicher Pathogene in einer Probe mittels einer Multiplex-PCR-Untersuchung. Weiterhin betrifft die Erfindung ein Kit zum gleichzeitigen Nachweis einer Mehrzahl unterschiedlicher Pathogene mittels einer Multiplex-PCR-Untersuchung in einer Probe.

Aus dem Stand der Technik sind Untersuchungen von Proben zum Nachweis von Pathogenen bekannt. So offenbart beispielsweise die Druckschrift WO 2016 / 164 407 A2 ein Verfahren zum Nachweis einer Mehrzahl von Pathogenen in einer Probe. Die Pathogene werden in einem Voranreicherungsmedium vermehrt. Anschließend werden in einem ersten Lyseschritt und in einem zweiten Lyseschritt Zellwände aufgebrochen, um ein Lysat herzustellen, welches geeignet ist, mit Hilfe eines Multiplex-PCR-Verfahrens untersucht zu werden. Der zweite Lyseschritt findet hierbei bei einer höheren Temperatur statt als der erste Schritt.

Es ist eine Aufgabe der vorliegenden Erfindung, ein Verfahren bereitzustellen, welches einen im Vergleich zum Stand der Technik verbesserten Ablauf bietet und ein Kit bereitzustellen, mit welchem ein solches Verfahren durchführbar ist.

Diese Aufgabe wird gelöst durch ein Verfahren gemäß Anspruch 1 und ein Kit gemäß Anspruch 10.

Zum Durchführen des Verfahrens wird vorzugsweise eine Probe aus einem zu untersuchenden Stoff entnommen. Denkbar ist, dass dieser Stoff ein Lebensmittel ist. Beispielsweise, aber nicht einschränkend, ist ferner denkbar, dass dieser Stoff ein Fleisch- oder Milcherzeugnis ist. Ein Fleischerzeugnis kann beispielsweise Hackfleisch, aufgeschnittenes Fleisch, Wurst, Schinken oder jedes andere Lebensmittel sein, in welchem Fleisch verarbeitet ist. Ein Milcherzeugnis kann beispielsweise Milch, Quark, Joghurt oder jedes andere Lebensmittel sein, in welchem Milch verarbeitet ist. Denkbar ist ferner, dass der Stoff Tiernahrung ist.

Vorzugsweise ist vorgesehen, dass die Probe mehr als 1 g und weniger als 100 g, besonders bevorzugt mehr als 10 g und weniger als 50 g und insbesondere 25 g schwer ist.

Zum Nachweis der Mehrzahl unterschiedlicher Pathogene durchläuft die Probe erfindungsgemäß zunächst einen Voranreicherungsschritt zum Voranreichern in einem Voranreicherungsmedium. Anschließend werden in einem Lyseschritt die Zellwände der Pathogene in der vorangereicherten Probe aufgebrochen. Dies dient dazu, die DNA der Pathogene freizulegen. Das Aufbrechen der Zellwände der Pathogene wird auch als Lyse bezeichnet. Die Lyse kann auf unterschiedliche Arten bewirkt werden. So können die Zellwände bei einer thermischen Lyse mit einer erhöhten Temperatur aufgebrochen werden. Bei einer chemischen Lyse geschieht der Zellaufschluss beispielsweise durch die Zugabe von chaotropen Salzen, Detergenzien oder anderen chemischen Hilfsmitteln. Eine enzymatische Lyse verwendet ein Enzym, beispielsweise Proteinase-K und/oder Lysozym zum Zellaufschluss. Proteinase-K trägt zudem auch zum Ausschalten von Nukleasen bei, welche beim Zellaufschluss freigesetzt werden. Möglich ist auch eine mechanische Lyse, bei der der Zellaufschluss beispielsweise durch mechanische Beanspruchung der Zellwände mit sogenannten Beads bewirkt wird. Beads sind Kügelchen aus einem harten Material, beispielsweise auf Keramik-, Glas- oder Zirkoniumbasis. Insbesondere ist vorgesehen, dass im Lyseschritt zunächst eine thermische Lyse und anschließend eine chemische Lyse zum Zellaufschluss verwendet wird. Zusätzlich kann eine enzymatische Lyse und/oder eine mechanische Lyse im Lyseschritt zum Zellaufschluss beitragen.

Ist der Lyseschritt abgeschlossen, so wird in einem Nachweisschritt mittels einer Multiplex-PCR-Untersuchung die Mehrzahl der Pathogene nachgewiesen. Hierzu wird das Lysat zu einem PCR-Reagenz hinzugegeben. Das PCR-Reagenz umfasst für jeden nachzuweisenden Zielorganismus, also für jedes nachzuweisende Pathogen, mindestens ein spezifisches Olignukleotid-Primerpaar (kurz: "Primer"), mindestens ein markiertes Oligonukleotid, welches als Sonde zum Nachweis der Pathogene genutzt werden kann, und eine hitzestabile DNA-Polymerase, beispielsweise eine Taq-Polymerase. Ferner umfasst das PCR-Reagenz Desoxy-Nukleotide, Cofaktoren für die Polymersase sowie eine Polymerase-Pufferlösung. In einem Thermocycler wird das Lysat zunächst auf etwa 96 °C erhitzt, so dass die doppelsträngige DNA in zwei Einzelstränge getrennt wird. Anschließend hybridisieren die Primer bei einer niedrigeren Temperatur an die Einzelstränge und definieren jeweils einen Startpunkt für die folgende DNA-Synthese. Für die DNA-Synthese wird die Temperatur wiederum erhöht. Hierbei füllt die Taq-Polymerase die freien Stränge mit Nukleotiden auf. Denkbar ist aber auch, dass die Temperatur für die DNA-Synthese nicht erhöht wird und dass die DNA-Synthese bei der gleichen Temperatur geschieht, wie das Hybridisieren der Primer. Der beschriebene Vorgang wird für eine festgelegte Anzahl von Zyklen wiederholt. Werden nach den festgelegten Zyklen keine Pathogene nachgewiesen, ist davon auszugehen, dass die Probe frei von den nachzuweisenden Pathogenen ist.

Um die Taq-Polymerase zu schützen und eine PCR-Untersuchung durchführen zu können, wird der Lyseschritt vor dem Nachweisschritt abgebrochen. Aus dem Stand der Technik ist bekannt, eine enzymatische Lyse durch eine Erhöhung der Temperatur abzubrechen. Wird mit einem Enzym, beispielsweise mit einer Proteinnase-K, lysiert, so kann das Enzym durch die Erhöhung der Temperatur deaktiviert werden. Die mit der Temperaturerhöhung verbundenen Nachteile, insbesondere Wartezeiten, werden bei dem erfindungsgemäßen Verfahren umgangen.

Denkbar ist, dass das Verfahren als Pooltesting durchgeführt wird, dass die Multiplex-PCR-Untersuchung also an einer PCR-Probe durchgeführt wird, die mehrere Lysate umfasst. Hierzu ist denkbar, dass die Lysate aus unterschiedlichen Proben hergestellt werden. Vorzugsweise ist hierzu vorgesehen, dass die unterschiedlichen Proben Proben aus unterschiedlichen Lebensmitteln sind. Denkbar ist aber auch, dass die unterschiedlichen Proben Proben aus gleichen Lebensmitteln unterschiedlicher Chargen sind.

Vorteilhafte Ausgestaltungen und Weiterbildungen der Erfindung sind den Unteransprüchen, sowie der Beschreibung unter Bezugnahme auf die Zeichnungen entnehmbar.

Gemäß einer bevorzugten Ausführungsform der Erfindung ist vorgesehen, dass der Abbruch der Lyse durch eine Verdünnung des Lysats unterstützt und/oder bewirkt wird und die Multiplex-PCR-Untersuchung an dem verdünnten Lysat durchgeführt wird. Hierdurch ist es auf vorteilhafte Weise möglich, die Lyse bei gleichbleibender oder sogar sinkender Temperatur abzubrechen. Denkbar ist, dass zum Verdünnen Nukleinsäure-freies und Nuclease-freies Wasser zum Lysat hinzu pipettiert wird. Hinzu pipettiert im Sinne der vorliegenden Erfindung ist so zu verstehen, dass das Nukleinsäure-freie und Nuclease-freie Wasser zum Lysat hinzugefügt wird oder dass das Lysat zum Nukleinsäure-freien und Nucleasefreien Wasser hinzugefügt wird. Vorzugsweise ist vorgesehen, dass die Verdünnung eine Verdünnung von 1:3 bis 1:100, besonders bevorzugt 1:10 bis 1:75 und insbesondere 1:20 bis 1:40 ist. Denkbar ist auch, dass ein in lypohilisierter Form vorliegendes PCR-Reagenz in Nukleinsäure- und Nuklease freiem Wasser gelöst wird und das Lysat anschließend zu pipettiert wird, so dass die genannte Verdüünung des Lysates erreicht wird. Es hat sich überraschend gezeigt, dass eine Verdünnung zu einem Abbruch der Lyse führt.

Alternativ oder zusätzlich ist vorzugsweise vorgesehen, dass der Abbruch der Lyse, insbesondere einer durch eine Protease vermittelten oder unterstützten Lyse, durch Hinzugabe eines Proteasehemmers, beispielsweise 4-(2-Aminoethyl)-benzolsulfonylfluorid-hydrochlorid (AEBSF) zum Lysat unterstützt und/oder bewirkt wird. Es hat sich überraschend gezeigt, dass die Hinzugabe von Proteasehemmer zum Lysat die Lyse stoppt. Wird der Lyseschritt unter Zuhilfenahme eines Enzyms durchgeführt, so wird das Enzym durch die Hinzugabe des Proteasehemmers zum Abbruch der Lyse chemisch deaktiviert. Vorzugsweise ist vorgesehen, dass zwischen den Lyseschritt und den Nachweisschritt eine Wartezeit von 1 Minute bis 40 Minuten eingehalten wird. Hierdurch wird sichergestellt, dass die Lyse abgebrochen wird, bevor das Lysat mit dem PCR-Reagenz in Kontakt kommt.

Gemäß einer weiteren bevorzugten Ausführungsform der vorliegenden Erfindung ist vorgesehen, dass der Abbruch der Lyse durch Hinzugabe eines Proteasehemmers, beispielsweise 4-(2-Aminoethyl)-benzolsulfonylfluorid-hydrochlorid (AEBSF), zum Lysat und einem Verdünnen unterstützt und/oder bewirkt wird. Vorzugsweise ist vorgesehen, dass das Verdünnen der Hinzugabe des Proteasehemmers zeitlich folgt. Die Multiplex-PCR-Untersuchung wird anschließend an dem verdünnten Lysat durchgeführt wird. Die Verdünnung ist vorzugsweise eine Verdünnung von 1:3 bis 1:100, besonders bevorzugt 1:10 bis 1:75 und insbesondere 1:20 bis 1:40. Die Hinzugabe des Proteasehemmers und die anschließende Verdünnung bewirken einen besonders sicheren Abbruch der Lyse. Wird der Lyseschritt unter Zuhilfenahme einer enzymatischen Lyse mit einem Enzym durchgeführt, so wird das Enzym durch die Hinzugabe des Proteasehemmers zum Abbruch der Lyse chemisch deaktiviert.

Hierzu ist vorzugsweise vorgesehen, dass zwischen der Hinzugabe des Proteasehemmers und dem Verdünnen eine Zuwartezeit eingehalten wird. Hierdurch wird dem Proteasehemmer ausreichend Zeit gegeben, die enzymatische Lyse chemisch abzubrechen.

Gemäß einer besonders bevorzugten Ausführungsform der vorliegenden Erfindung ist vorgesehen, dass das Lysat ein Rohlysat ist. Ein Rohlysat ist ein Lysat, bei welchem keine Trennung von DNA und Reststoffen aus dem Lysat durchgeführt wurde. Eine Konzentrierung von DNA erfolgt dabei nicht. Hierdurch entfällt auf vorteilhafte Weise eine Aufreinigung des Lysats. Das Verfahren ist somit deutlich zeitsparender und weniger aufwendig und mittelsparender im Vergleich zu Verfahren, bei denen eine Reinigung bzw. Konzentrierung unter Zuhilfenahme von spin columns oder magnetic beads bedienen. Mit Trennung kann eine physikalische Trennung gemeint sein. Denkbar ist aber auch, dass mit Trennung eine Bindung der DNA an eine Oberfläche, beispielsweise eine Oberfläche der Beads oder einer Säulenmatrix, gemeint ist.

Zur Verbesserung der Effektivität der Lyse ist gemäß einer bevorzugten Ausführungsform der vorliegenden Erfindung vorgesehen, dass während des Lyseschrittes zunächst eine thermische Lyse bei einer ersten Temperatur durchgeführt wird. Die Zellwände der Pathogene werden also zunächst durch Erhitzen aufgebrochen. Anschließend werden eine chemische Lyse und/oder eine enzymatische Lyse und/oder eine mechanische Lyse bei einer zweiten Temperatur durchgeführt. Die zweite Temperatur ist niedriger als die erste Temperatur, die Temperatur wird also nach der thermischen Lyse zum Start der chemischen bzw. enzymatischen bzw. mechanische Lyse abgesenkt. Durch die Kombination von thermischer Lyse, chemischer und enzymatischer bzw. mechanischer Lyse werden die Zellwände der Pathogene sehr sicher aufgebrochen. Denkbar ist, dass die enzymatische Lyse mit einer Proteinnase-K durchgeführt wird. Denkbar ist, dass die mechanische Lyse mit Beads, beispielsweise auf Keramik-, Glas- oder Zirkoniumbasis, wird.

Gemäß einer weiteren bevorzugten Ausführungsform der vorliegenden Erfindung ist vorgesehen, dass das Voranreicherungsmedium ein nicht-selektives Voranreicherungsmedium ist und dass die Voranreicherung eine nicht-selektive Voranreicherung ist. Das nicht-selektive Voranreicherungsmedium weist beispielsweise keine Antibiotika auf. Somit werden die unterschiedlichen Pathogene und in der Probe gegebenenfalls enthaltene pathogene oder nicht-pathogene Begleitflora gleichzeitig vermehrt. Eine Begleitflora im Sinne der vorliegenden Erfindung umfasst Mikroorganismen, welche nicht zu der Mehrzahl von Pathogenen gehören.

Alternativ hierzu ist vorzugsweise vorgesehen, dass das Voranreicherungsmedium ein selektives Voranreicherungsmedium ist und dass die Voranreicherung eine selektive Voranreicherung ist.

Insbesondere ist vorgesehen, dass die Mehrzahl unterschiedlicher Pathogene mindestens zwei Pathogene aus der folgenden Gruppe von Pathogenen aufweist: Familie der Enterobacteriaceae, Gattung Salmonella, Gattung Listeria, Gattung Staphylococcus und Shigatoxin bildende E.coli, und Gattung Cronobacter. Vorzugsweise ist vorgesehen, dass die Mehrzahl unterschiedlicher Pathogene die Familie der Enterobacteriaceae, die Gattung Salmonella, die Gattung Listeria, die Gattung Staphylococcus und Shigatoxin bildende E.coli aufweist. Somit ist es möglich, für Lebensmittel gesetzlich vorgeschriebene Untersuchungen auf Pathogene zeitsparend gleichzeitig durchzuführen.

Ein weiterer Gegenstand der vorliegenden Erfindung zum Lösen der eingangsgestellten Aufgabe ist ein Kit nach Anspruch 10. Das erfindungsgemäße Kit zum Nachweis einer Mehrzahl unterschiedlicher Pathogene ist zur Verwendung mit dem erfindungsgemäßen Verfahren vorgesehen. Das erfindungsgemäße Kit weist das Voranreicherungsmedium zum Voranreichern im Voranreicherungsschritt auf. Weiterhin weist das erfindungsgemäße Kit ein Lysereagenz zum Aufbrechen der Zellwände im Lyseschritt auf. Das Lysereagenz weist vorzugsweise Proteinase-K auf. Ferner umfasst das erfindungsgemäße Kit ein PCR-Reagenz zum Durchführen der Multiplex-PCR-Untersuchung im Nachweisschritt.

Gemäß einer bevorzugten Ausführungsform der vorliegenden Erfindung ist vorgesehen, dass das Kit einen Proteasehemmer, beispielsweise 4-(2-Aminoethyl)-benzolsulfonylfluorid-hydrochlorid (AEBSF), zum Abbruch der Lyse umfasst. Denkbar ist ferner, dass das Kit einen Lysepuffer umfasst.

Vorzugsweise ist vorgesehen, dass das Kit Nukleinsäure-freies und Nuclease-freies Wasser zum Verdünnen des Lysats umfasst.

Vorzugsweise ist vorgesehen, dass die Mehrzahl unterschiedlicher Pathogene Salmonalla spp., Listeria spp., Listeria monocytogenes, Enterobactereacea umfasst. Vorzugsweise ist vorgesehen, dass die Mehrzahl unterschiedlicher Pathogene Cronobater, Coagolase posive Staphylokokken, STX/STC und EAC umfasst.

Alle zuvor im Zusammenhang mit dem erfindungsgemäßen Verfahren offenbarten Einzelheiten, Merkmale und Vorteile beziehen sich gleichfalls auf das erfindungsgemäße Kit.

Weitere Einzelheiten, Merkmale und Vorteile der Erfindung ergeben sich aus der Zeichnung sowie aus der nachfolgenden Beschreibung von bevorzugten Ausführungsformen anhand der Zeichnung. Die Zeichnung illustriert dabei lediglich eine beispielhafte Ausführungsform der Erfindung, welche den Erfindungsgedanken nicht einschränkt.

Es zeigt:

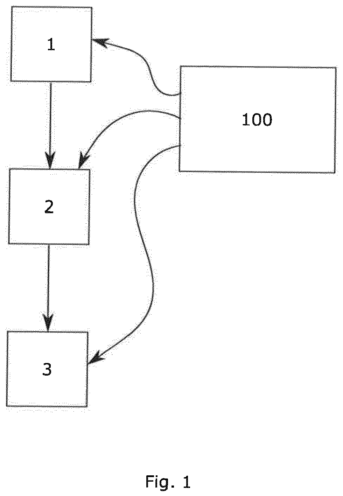

Fig. 1: einen Verfahrensablauf gemäß einer bevorzugten Ausführungsform der vorliegenden Erfindung mit einem Kit gemäß einer bevorzugten Ausführungsform der vorliegenden Erfindung in schematischer Darstellung.

Figur 1 zeigt schematisch ein Verfahren gemäß einer bevorzugten Ausführungsform der vorliegenden Erfindung. Weiterhin zeigt Figur 1 ein Kit 100 gemäß einer bevorzugten Ausführungsform der vorliegenden Erfindung stark schematischer Abbildung.

In dem gezeigten Verfahren zum Nachweis einer Mehrzahl unterschiedlicher Pathogene in einer Probe wird zunächst in einem Voranreicherungsschritt 1 die Probe zum Voranreichern in ein Voranreicherungsmedium gegeben. Die Probe ist eine 25 g schwere Lebensmittelprobe, welche in 225 ml des Voranreicherungsmediums bei 37 °C 18 bis 30 Stunden, insbesondere 22 +/- 2 Stunden lang inkubiert wird. Das Voranreicherungsmedium ist hier ein nicht-selektives Voranreicherungsmedium zur nicht-selektiven Voranreicherung mehrerer Pathogene.

Nach ausreichender Voranreicherung werden in einem Lyseschritt 2 die Zellwände der Pathogene in der vorangereicherten Probe aufgebrochen. Hierzu wird die vorangereicherte Probe zunächst auf eine Temperatur von 95 °C +/- 5 °C erhitzt und einer thermischen Lyse unterzogen. Anschließend wird die Temperatur abgesenkt und Reagenzen für eine chemische Lyse hinzugegeben. Denkbar ist, dass ferner ein Enzym, beispielsweise Proteinnase-K, zur Bewirkung einer enzymatischen Lyse hinzugegeben wird. Weiterhin ist denkbar, dass die Lyse zusätzlich oder alternativ durch eine mechanische Lyse bewirkt oder unterstützt wird. Die enzymatische Lyse wird nach einer Inkubationszeit von 5 bis 30 Minuten abgebrochen. Dies geschieht unter Beibehaltung oder Absenkung der Temperatur. Hierzu wird dem Lysat ein Proteasehemmer zum Deaktivieren der Proteinnase-K hinzugegeben. Weiterhin wird das Lysat nach dem Hinzugegeben des Proteasehemmers und einer Zuwartezeit mit Nukleinsäure-freiem und Nucleasefreiem Wasser verdünnt. Die Verdünnung beträgt hierbei vorzugsweise 1:20 bis 1:40.

In der gezeigten beispielhaften Ausführungsform ist vorgesehen, dass das Lysat ein Rohlysat ist.

Ist die Lyse abgebrochen, der Lyseschritt 2 also beendet, werden die Pathogene in einem Nachweisschritt 3 mittels einer Multiplex-PCR-Untersuchung nachgewiesen. Hierzu wird einem PCR-Reagenz das verdünnte Rohlysat hinzugefügt. Das PCR-Reagenz umfasst für jeden nachzuweisenden Zielorganismus, also für jedes nachzuweisende Pathogen, mindestens ein spezifisches Olignukleotid-Primerpaar (kurz: "Primer"), mindestens ein markiertes Oligonukleotid, welches als Sonde zum Nachweis der Pathogene genutzt werden kann, und eine hitzestabile DNA-Polymerase, beispielsweise eine Taq-Polymerase. Ferner umfasst das PCR-Reagenz Desoxy-Nukleotide, Cofaktoren für die Polymerase sowie eine Polymerase-Pufferlösung.. In einem Thermocycler wird das Lysat zunächst auf etwa 96 °C erhitzt, so dass die doppelsträngige DNA in zwei Einzelstränge getrennt wird. Anschließend hybridisieren die Primer bei einer niedrigeren Temperatur an die Einzelstränge und definieren jeweils einen Startpunkt für die folgende DNA-Synthese. Für die DNA-Synthese wird die Temperatur wiederum erhöht. Hierbei füllt die Taq-Polymerase die freien Stränge mit Nukleotiden auf. Denkbar ist aber auch, dass die Temperatur für die DNA-Synthese nicht erhöht wird und dass die DNA-Synthese bei der gleichen Temperatur geschieht, wie das Hybridisieren der Primer. Der beschriebene Vorgang wird für eine festgelegte Anzahl von Zyklen wiederholt. Werden nach den festgelegten Zyklen keine Pathogene nachgewiesen, ist davon auszugehen, dass die Probe frei von den nachzuweisenden Pathogenen ist.

Das erfindungsgemäße Kit 100 umfasst hierbei das nicht-selektive Voranreicherungsmedium, ein Lysereagenz, beispielsweise mit Proteinase-K, vorzugsweise einen Lysepuffer, einen Proteasehemmer zum Abbruch der enzymatischen Lyse, Nukleinsäure-freies und Nuclease-freiesWasser zum Verdünnen des Rohlysats sowie das PCR-Reagenz.

### Bezugszeichenliste:

- 1: Voranreicherungsschritt
- 2: Lyseschritt
- 3: Nachweisschritt
- 100: Kit

## Patentansprüche

1. Verfahren zum Nachweis einer Mehrzahl unterschiedlicher Pathogene in einer Probe, wobei
in einem Voranreicherungsschritt (1) die Probe zum Voranreichern in ein Voranreicherungsmedium gegeben wird,
in einem Lyseschritt (2) Zellwände der Pathogene in der vorangereicherten Probe aufgebrochen werden und somit ein Lysat hergestellt wird,
in einem Nachweisschritt (3) mittels einer Multiplex-PCR-Untersuchung die Mehrzahl der Pathogene nachgewiesen wird,
wobei der Voranreicherungsschritt (1), der Lyseschritt (2) und der Nachweisschritt (3) in dieser Reihenfolge durchlaufen werden und wobei die Lyse ohne eine Erhöhung der Temperatur vor dem Nachweisschritt (3) abgebrochen wird.

2. Verfahren nach Anspruch 1, **dadurch gekennzeichnet, dass** der Abbruch der Lyse durch eine Verdünnung des Lysats unterstützt und/oder bewirkt wird und die Multiplex-PCR-Untersuchung an dem verdünnten Lysat durchgeführt wird, wobei die Verdünnung vorzugsweise eine Verdünnung von 1:3 bis 1:100, besonders bevorzugt 1:10 bis 1:75 und insbesondere 1:10 bis 1:40 ist.

3. Verfahren nach einem der vorhergehenden Ansprüche, **dadurch gekennzeichnet, dass** der Abbruch Lyse durch Hinzugabe von eines Proteasehemmers, vorzugsweise von 4-(2-Aminoethyl)-benzolsulfonylfluorid-hydrochlorid, zum Lysat unterstützt und/oder bewirkt wird.

4. Verfahren nach Anspruch 1, **dadurch gekennzeichnet, dass** der Abbruch der Lyse durch Hinzugabe eines Proteasehemmers, vorzugsweise von 4-(2-Aminoethyl)-benzolsulfonylfluorid-hydrochlorid zum Lysat und anschließenden Verdünnen unterstützt und/oder bewirkt wird und die Multiplex-PCR-Untersuchung an dem verdünnten Lysat durchgeführt wird, wobei die Verdünnung vorzugsweise eine Verdünnung von 1:3 bis 1:100, besonders bevorzugt 1:10 bis 1:75 und insbesondere 1:10 bis 1:40 ist.

5. Verfahren nach Anspruch 4, **dadurch gekennzeichnet, dass** zwischen der Hinzugabe des Proteasehemmers und dem Verdünnen eine Zuwartezeit eingehalten wird.

6. Verfahren nach einem der vorhergehenden Ansprüche, **dadurch gekennzeichnet, dass** das Lysat ein Rohlysat ist.

7. Verfahren nach einem der vorhergehenden Ansprüche, **dadurch gekennzeichnet, dass** während des Lyseschrittes (2) zunächst eine thermische Lyse bei einer ersten Temperatur durchgeführt wird und anschließend eine chemische Lyse und/oder eine enzymatische Lyse und/oder mechanische Lyse bei einer zweiten Temperatur durchgeführt wird, wobei die zweite Temperatur niedriger ist als die erste Temperatur.

8. Verfahren nach einem der vorhergehenden Ansprüche, **dadurch gekennzeichnet, dass** das Voranreicherungsmedium ein nicht-selektives Voranreicherungsmedium ist und dass die Voranreicherung eine nicht-selektive Voranreicherung ist.

9. Verfahren nach einem der vorhergehenden Ansprüche, **dadurch gekennzeichnet, dass** die Mehrzahl unterschiedlicher Pathogene mindestens zwei Pathogene aus der folgenden Gruppe von Pathogenen aufweist: Familie der Enterobacteriaceae, Gattung Salmonella, Gattung Listeria, Gattung Staphylococcus und Shigatoxin bildende E.coli, und Gattung Cronobacter, wobei die Mehrzahl unterschiedlicher Pathogene vorzugsweise die Familie der Enterobacteriaceae, die Gattung Salmonella, die Gattung Listeria, die Gattung Staphylococcus und Shigatoxin bildende E.coliaufweist.

10. Kit (100) zum Nachweis einer Mehrzahl unterschiedlicher Pathogene mit einem Verfahren nach einem der vorhergehenden Ansprüche, wobei das Kit folgende Komponenten aufweist:
- Voranreicherungsmedium zum Voranreichern in Voranreicherungsschritt (1),
- Lysereagenz zum Aufbrechen der Zellwände im Lyseschritt (2),
- ein PCR-Reagenz zum Durchführen der Multiplex-PCR-Untersuchung im Nachweisschritt (3).

11. Kit (100) nach Anspruch 10, **dadurch gekennzeichnet, dass** das Kit (100) einen Proteasehemmer, beispielsweise 4-(2-Aminoethyl)-benzolsulfonylfluorid-hydrochlorid (AEBSF), zum Abbruch der Lyse umfasst.

12. Kit (100) nach einem der Ansprüche 10 bis 11, **dadurch gekennzeichnet, dass** das Kit Nukleinsäure-freies und Nuclease-freies Wasser zum Verdünnen des Lysats umfasst.
